# EUROPÄISCHE PATENTSCHRIFT

(11) **EP 1 519 926 B1**
(45) Veröffentlichungstag und Bekanntmachung des Hinweises auf die Patenterteilung: **02.11.2005**
(21) Anmeldenummer: 03732578.4
(22) Anmeldetag: 17.06.2003
(51) Int. Cl.: C07D 275/03

(54) **VERFAHREN ZUR HERSTELLUNG VON 3,4-DICHLOR-N-(2-CYANO-PHENYL)-5-ISOTHIAZOLCARBOXAMID**
METHOD FOR PRODUCING 3,4-DICHLORO-N-(2-CYANO-PHENYL)-5-ISOTHIAZOLE CARBOXAMIDE
PROCEDE DE PRODUCTION DE 3,4-DICHLORO-N-(2-CYANO-PHENYL)-5-ISOTHIAZOLCARBOXAMIDE

(30) Priorität: 27.06.2002 DE 10228732
(43) Veröffentlichungstag der Anmeldung: 06.04.2005
(73) Patentinhaber: Bayer CropScience AG, 40789 Monheim (DE)
(72) Erfinder: HIMMLER, Thomas, 51519 Odenthal (DE)
(86) Internationale Anmeldenummer: PCT/EP2003/006360
(87) Internationale Veröffentlichungsnummer: WO 2004/002968

(56) Entgegenhaltungen:
- WO-A-99/24413

## Beschreibung

Die vorliegende Erfindung betrifft ein neues Verfahren zur Herstellung des bekannten 3,4-Dichlor-N-(2-cyano-phenyl)-5-isothiazolcarboxamids, das als Wirkstoff mit mikrobiziden Eigenschaften verwendbar ist.

Es ist bereits bekannt geworden, dass man 3,4-Dichlor-N-(2-cyano-phenyl)-5-isothiazolcarboxamid erhält, wenn man 3,4-Dichlor-isothiazol-5-carbonsäurechlorid mit 2-Cyano-anilin umsetzt (vgl. WO 99-24 413). Die Ausbeute bei dieser Herstellungsmethode ist hoch. Nachteilig ist aber, dass das als Ausgangsprodukt benötigte 2-Cyano-anilin nur durch eine aufwendige Synthese zugänglich ist (vgl. DE-A 2 115 624 und DE-A 2 115 625). So ist es erforderlich, zunächst Anthranilsäureamid in Gegenwart von Dimethylformamid mit Phosgen umzusetzen und das dabei entstehende N-2-Cyano-phenyl-N',N'-dimethyl-formamidinium-Hydrochlorid dann in einem zweiten Schritt mit Natriumacetat in wässrigem Medium zu behandeln.

Es wurde nun gefunden, dass man 3,4-Dichlor-N-(2-cyano-phenyl)-5-isothiazolcarboxamid der Formel 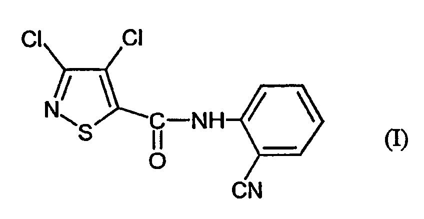 erhält, indem man
a) 3,4-Dichlor-isothiazol-5-carbonsäurechlorid der Formel 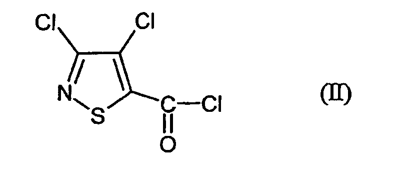 mit Anthranilsäureamid der Formel 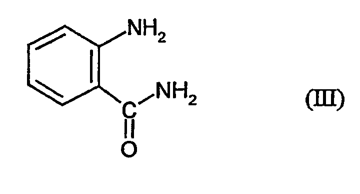 in Gegenwart eines Säureakzeptors und in Gegenwart eines aprotischen Verdünnungsmittels umsetzt und
b) dann das entstandene N-[2-(Aminocarbonyl)-phenyl]-3,4-dichlor-5-isothiazolcarboxamid der Formel 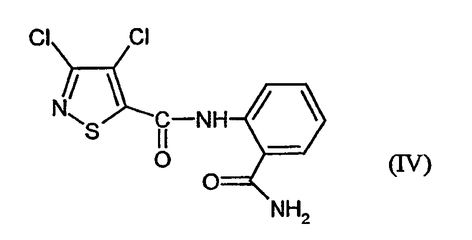 mit einem Dehydratisierungsmittel gegebenenfalls in Gegenwart eines zusätzlichen aprotischen Verdünnungsmittels umsetzt.

Es ist als ausgesprochen überraschend zu bezeichnen, dass sich 3,4-Dichlor-N-(2-Cyano-phenyl)-5-isothiazolcarboxamid der Formel (I) nach dem erfindungsgemäßen Verfahren in glatter Reaktion ohne störende Nebenreaktionen herstellen lässt. So musste aufgrund des vorbekannten Standes der Technik damit gerechnet werden, dass N-acylierte Anthranilsäurederivate unter sauren, basischen oder neutralen Bedingungen unter Abspaltung von Wasser leicht zu Benzoxazinonen beziehungsweise Chinazolinolen oder Chinazolinonen cyclisiert werden (vgl. Formaco Ed. Sci. 39 (1984), 120; J. Heterocycl. Chem. 16 (1979) 711; J. prakt.Chem. 111 (1925) 48 und Egypt. J. Chem. 31 (1988) 241). Im Gegensatz zu den Erwartungen wird die erfindungsgemäße Umsetzung aber nicht durch derartige unerwünschte Kondensationen beeinträchtigt.

Das erfindungsgemäße Verfahren zeichnet sich durch eine Reihe von Vorteilen aus. So ermöglicht es die Synthese des 3,4-Dichlor-N-(2-cyano-phenyl)-5-isothiazolcarboxamides der Formel (I) in extrem hoher Ausbeute und hervorragender Reinheit. Günstig ist auch, dass die Durchführung der Umsetzung und die Isolierung des Reaktionsproduktes keinerlei Schwierigkeiten bereitet. Außerdem lässt sich das erfindungsgemäße Verfahren problemlos in den technischen Maßstab übertragen.

Verwendet man bei der Durchführung des erfindungsgemäßen Verfahrens in der ersten Stufe Triethylamin als Säureakzeptor und in der zweiten Stufe Thionylchlorid im Gemisch mit Dimethylformamid als Dehydratisierungsmittel, so kann der Verlauf der Umsetzung durch das folgende Formelschema veranschaulicht werden.

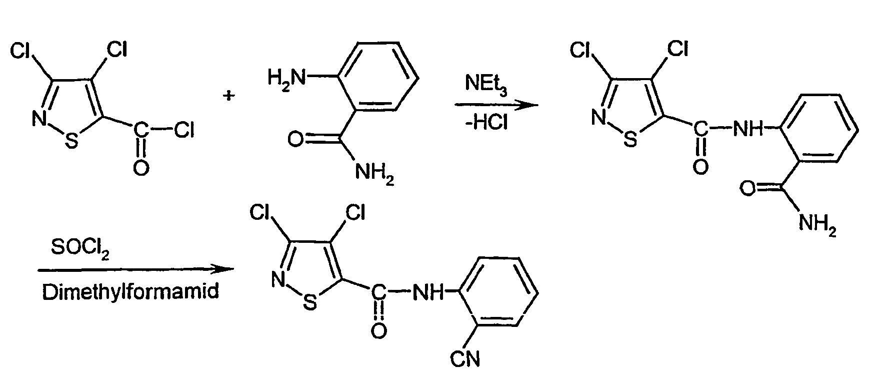

Das bei der Durchführung des erfindungsgemäßen Verfahrens als Ausgangsprodukt benötigte 3,4-Dichlor-isothiazol-5-carbonsäurechlorid der Formel (II) ist bekannt (vgl. US-A 5 240 951).

Ebenso ist das als Reaktionskomponente benötigte Anthranilsäureamid bekannt (vgl. DE-A 2 115 625).

Als Säureakzeptoren kommen bei der Durchführung der ersten Stufe des erfindungsgemäßen Verfahrens vorzugsweise tertiäre Amine in Frage. Beispielhaft genannt seien Trimethylamin, Triethylamin, Tributylamin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel können bei der Durchführung der ersten Stufe des erfindungsgemäßen Verfahrens alle üblichen aprotischen, organischen Solventien eingesetzt werden. Vorzugsweise verwendbar sind gegebenenfalls halogenierte aromatische Kohlenwasserstoffe, wie Toluol oder Chlorbenzol, ferner chlorierte Kohlenwasserstoffe, wie Dichlormethan, Chloroform, Tetrachlorkohlenstoff, Dichlorethan oder Trichlorethan, und weiterhin Amide, wie Dimethylformamid und Dimethylacetamid. Besonders bevorzugt ist Dimethylformamid.

Das bei der Durchführung der ersten Stufe des erfindungsgemäßen Verfahrens anfallende N-[2-(Aminocarbonyl)-phenyl]-3,4-dichlor-5-isothiazolcarboxamid der Formel (IV) ist bisher noch nicht beschrieben worden.

Als Dehydratisierungsmittel kommen bei der Durchführung der zweiten Stufe des erfindungsgemäßen Verfahrens alle diejenigen Reagenzien in Frage, die zur Abspaltung von Wasser aus Amiden geeignet sind. Vorzugsweise verwendbar sind Gemische aus Dimethylformamid und Thionylchlorid, Phosphoroxychlorid, Phosgen oder Chlormethylen-dimethylammmoniumchlorid.

Als Verdünnungsmittel können bei der Durchführung der zweiten Stufe des erfindungsgemäßen Verfahrens alle üblichen aprotischen, organischen Solventien eingesetzt werden. Vorzugsweise verwendbar sind gegebenenfalls halogenierte aromatische Kohlenwasserstoffe, wie Toluol oder Chlorbenzol, ferner chlorierte Kohlenwasserstoffe, wie Dichlormethan, Chloroform, Tetrachlorkohlenstoff, Dichlorethan oder Trichlorethan, und weiterhin Amide, wie Dimethylformamid und Dimethylacetamid. Besonders bevorzugt ist Dimethylformamid.

Die Temperaturen können bei der Durchführung des erfindungsgemäßen Verfahrens innerhalb eines größeren Bereiches variiert werden. Im Allgemeinen arbeitet man bei der Durchführung der ersten Stufe bei Temperaturen zwischen 0°C und 160°C, vorzugsweise zwischen 0°C und 120°C. Bei der Durchführung der zweiten Stufe arbeitet man im Allgemeinen bei Temperaturen zwischen -20°C und +80°C, vorzugsweise zwischen 0°C und 60°C.

Bei der Durchführung des erfindungsgemäßen Verfahrens arbeitet man sowohl bei der Umsetzung in der ersten Stufe als auch der zweiten Stufe im Allgemeinen unter Atmosphärendruck. Es ist jedoch auch möglich, unter erhöhtem Druck zu arbeiten.

Bei der Durchführung der ersten Stufe des erfindungsgemäßen Verfahrens setzt man auf 1 Mol an 3,4-Dichlor-isothiazol-5-carbonsäurechlorid der Formel (II) im Allgemeinen zwischen 1 und 1,5 Mol an Anthranilsäureamid der Formel (III) sowie eine äquivalente Menge an Säureakzeptor ein. Die Aufarbeitung erfolgt nach üblichen Methoden. Im Allgemeinen geht man in der Weise vor, dass man das Reaktionsgemisch einengt, den verbleibenden Rückstand mit Wasser verrührt, absaugt, durch Behandlung mit einem geeigneten Lösungsmittel reinigt, erneut absaugt und trocknet.

Bei der Durchführung der zweiten Stufe des erfindungsgenmäßen Verfahrens setzt man auf 1 Mol an N-[2-(Aminocarbonyl)-phenyl]-3,4-dichlor-5-isothiazolcarboxamid der Formel (IV) im Allgemeinen zwischen 1 und 2 Mol an Dehydratisierungsmittel sowie gegebenenfalls einen Überschuss an Dimethylformamid ein. Die Aufarbeitung erfolgt nach üblichen Methoden. Im Allgemeinen geht man in der Weise vor, dass man das Reaktionsgemisch mit Wasser versetzt und den dabei anfallenden Feststoff absaugt, wäscht und trocknet.

In einer besonderen Variante kann das erfindungsgemäße Verfahren als Eintopf-Reaktion durchgeführt werden. Hierbei setzt man zunächst 3,4-Dichlor-isothiazol-5-carbonsäurechlorid der Formel (II) mit Anthranilsäureamid der Formel (III) in Gegenwart von Säureakzeptor um und fügt dann ohne vorherige Isolierung des N-[2-(Aminocarbonyl)-phenyl]-3,4-dichlor-isothiazolcarboxamids der Formel (IV) das Dehydratisierungsreagenz hinzu. Die Aufarbeitung erfolgt wiederum nach üblichen Methoden. Im Allgemeinen verfährt man in der bereits oben angegebenen Weise.

Das nach dem erfindungsgemäßen Verfahren erhältliche 3,4-Dichlor-N-(2-Cyanophenyl)-5-isothiazolcarboxamid der Formel (I) und dessen Verwendung zur Bekämpfung von unerwünschten Mikroorganismen sind bekannt (vgl. WO 99-24 413).

Das erfindungsgemäße Verfahren wird durch die folgenden Beispiele veranschaulicht.

### Beispiele

### Beispiel 1

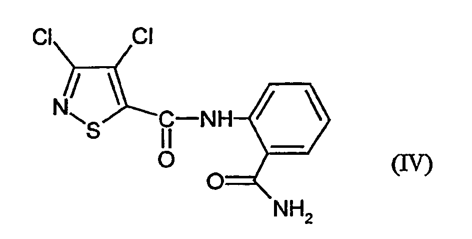

In ein Gemisch aus 15 ml Dimethylformamid, 3 g (22 mmol) Anthranilsäureamid und 2,23 g (22 mmol) Triethylamin wird bei 60°C unter Rühren eine Lösung von 4,33 g (22 mmol) 3,4-Dichlor-isothiazolcarbonsäurechlorid eingetropft. Danach wird das Reaktionsgemisch noch eine Stunde bei 60°C nachgerührt und dann unter vermindertem Druck eingeengt. Der verbleibende Rückstand wird mit 35 ml Wasser verrührt und anschließend abgesaugt. Der dabei anfallende, noch feuchte Feststoff wird mit Isopropanol aufgekocht, auf Raumtemperatur abgekühlt, abgesaugt und getrocknet.

Man erhält auf diese Weise 5,44 g eines weißen Feststoffes, der gemäß HPLC zu 100 % aus N-[2-(Aminocarbonyl)-phenyl]-3,4-dichlor-5-isothiazolcarboxamid besteht. Die Ausbeute errechnet sich danach zu 86,0 % der Theorie.
¹H-NMR(400 MHz, d-DMSO): δ = 7,27-7,31 (m; 1H), 7,58-7,63 (m; 1H), 7,88-7,94 (m; 2H), 8,44-8,50 (m; 2H), 12,80 (s: 1H) ppm.
Schmelzpunkt: 244-248°C.

### Beispiel 2

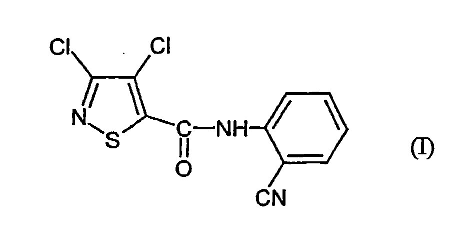

Zu einer Suspension von 0,79 g (2,5 mmol) N-[2-(Aminocarbonyl)-phenyl]-3,4-dichlor-5-isothiazolcarboxamid in 5 ml Dimethylformamid werden bei 60°C unter Rühren 0,416 g (3,5 mmol) Thionylchlorid tropfenweise hinzugegeben. Das Reaktionsgemisch wird noch 4 Stunden bei 60°C gerührt, dann auf Raumtemperatur abgekühlt, mit 5 ml Wasser versetzt und 15 Minuten gerührt. Der anfallende Feststoff wird abgesaugt, zweimal mit je 10 ml Wasser gewaschen und getrocknet.

Man erhält auf diese Weise 0,63 g eines weißen Feststoffes, der gemäß ¹H-NMR-Spektrum zu 91 % aus 3,4-Dichlor-N-(2-cyano-phenyl)-5-isothiazolcarboxamid besteht. Die Ausbeute errechnet sich danach zu 76,9 % der Theorie.
¹H-NMR(400 MHz, d-DMSO): δ = 7,47-7,51 (m; 1H), 7,71-7,77 (m; 1H) 7,79-7,81 (m; 1H), 7,92-7,94 (m; 1H), 11,05 (s; 1H) ppm.

### Beispiel 3

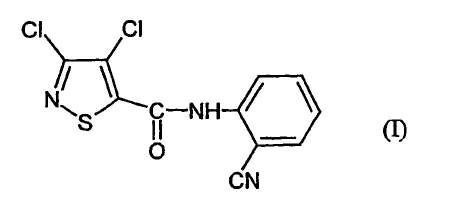

Zu einer Suspension von 3,16 g (10 mmol) N-[2-(Aminocarbonyl)-phenyl]-3,4-dichlor-5-isothiazolcarboxamid in 20 ml Dimethylformamid werden bei 0°C unter Rühren 1,67 g (14 mmol) Thionylchlorid tropfenweise hinzugegeben. Das Reaktionsgemisch wird noch 2 Stunden bei 0°C gerührt, dann unter Eiskühlung mit 25 ml Wasser versetzt und 15 Minuten gerührt. Der anfallende Feststoff wird abgesaugt, zweimal mit je 20 ml Wasser gewaschen und getrocknet.

Man erhält auf diese Weise 2,95 g eines weißen Feststoffes, der gemäß HPLC zu 99 % aus 3,4-Dichlor-N-(2-cyano-phenyl)-5-isothiazolcarboxamid besteht. Die Ausbeute errechnet sich danach zu 98 % der Theorie.

### Beispiel 4

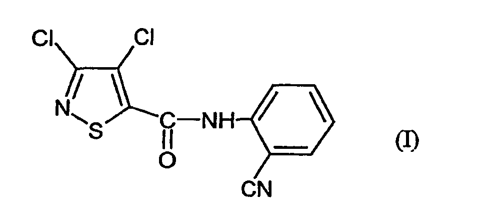

Zu einer Suspension von 1,58 g (5 mmol) N-[(2-Aminocarbonyl)-phenyl]-3,4-dichlor-5-isothiazolcarboxamid in 10 ml Dimethylformamid werden bei Raumtemperatur unter Rühren 0,9 g (7 mmol) Chlormethylendimethylammoniumchlorid hinzugegeben. Das Reaktionsgemisch wird anschließend eine Stunde bei 60°C gerührt, dann mit 25 ml Wasser versetzt und 15 Minuten gerührt. Der anfallende Feststoff wird abgesaugt, zweimal mit je 20 ml Wasser gewaschen und getrocknet.

Man erhält auf diese Weise 1,64 g eines weißen Feststoffes, der gemäß HPLC zu 88 % aus 3,4-Dichlor-N-(2-cyano-phenyl)-5-isothiazolcarboxamid besteht. Die Ausbeute errechnet sich danach zu 96 % der Theorie.

### Beispiel 5

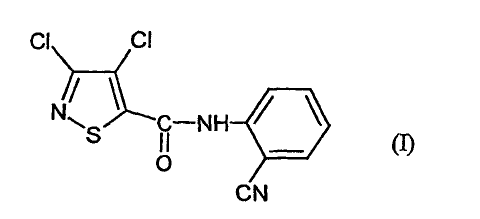

Zu einer Lösung von 6 g (44 mmol) Anthranilsäureamid und 4,45 g (44 mmol) Triethylamin in 24 g Dimethylformamid wird bei Raumtemperatur unter Rühren eine Lösung von 8,66 g (40 mmol) 3,4-Dichlor-isothiazol-5-carbonsäurechlorid in 20 g Dimethylformamid hinzugetropft. Nach beendeter Zugabe wird das Reaktionsgemisch eine Stunde bei 20°C gerührt. Zu der entstandenen Suspension werden bei Raumtemperatur 6,67 g (56 mmol) Thionylchlorid unter Rühren hinzugetropft. Das Reaktionsgemisch wird noch 2 Stunden bei Raumtemperatur nachgerührt, dann mit 80 ml Wasser versetzt und weitere 15 Minuten gerührt. Der anfallende Feststoff wird abgesaugt, zweimal mit je 40 ml Wasser gewaschen und getrocknet. Das erhaltene Produkt wird 30 Minuten in 30 ml Isopropanol gekocht. Nach dem Abkühlen auf Raumtemperatur wird der anfallende Feststoff abgesaugt, zweimal mit je 10 ml Isopropanol gewaschen und getrocknet. Man erhält auf diese Weise 11,48 g eines Feststoffes, der gemäß HPLC zu 96,75 % aus 3,4-Dichlor-N-(2-cyano-phenyl)-5-isothiazolcarboxamid besteht. Die Ausbeute über 2 Stufen errechnet sich danach zu 93,1 % der Theorie. Das entspricht einer Ausbeute von 96,5 % der Theorie pro Stufe.

### Beispiel 6

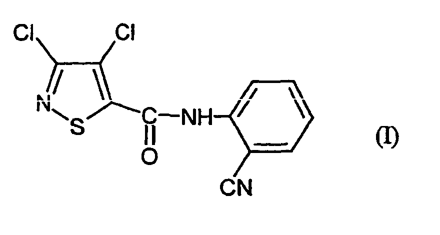

Zu einer Lösung von 6 g (44 mmol) Anthranilsäureamid und 4,45 g (44 mmol) Triethylamin in 24 g Dimethylformamid wird bei 0°C unter Rühren eine Lösung von 8,66 g (40 mmol) 3,4-Dichlor-isothiazol-5-carbonsäurechlorid in 20 g Dimethylformamid hinzugetropft. Nach beendeter Zugabe wird das Reaktionsgemisch 2 Stunden bei 0°C gerührt. Zu der entstandenen Suspension werden bei 0°C unter Rühren 6,67 g (56 mmol) Thionylchlorid hinzugetropft. Das Reaktionsgemisch wird noch 2 Stunden bei 0°C nachgerührt, dann mit 80 ml Wasser versetzt und weitere 15 Minuten gerührt. Der anfallende Feststoff wird abgesaugt, zweimal mit je 40 ml Wasser gewaschen und getrocknet. Das erhaltene Produkt wird 30 Minuten in 30 ml Isopropanol gekocht. Nach dem Abkühlen auf Raumtemperatur wird der anfallende Feststoff abgesaugt, zweimal mit je 10 ml Isopropanol gewaschen und getrocknet. Man erhält auf diese Weise 10,9 g eines Feststoffes, der gemäß HPLC zu 96,5 % aus 3,4-Dichlor-N-(2-cyano-phenyl)-5-isothiazolcarboxamid besteht. Die Ausbeute über 2 Stufen errechnet sich danach zu 90,0 % der Theorie.

### Beispiel 7

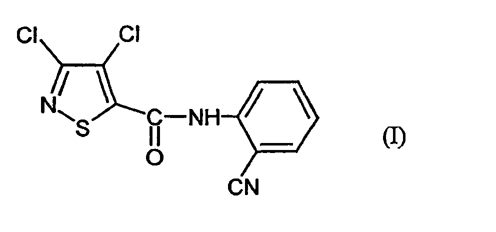

Zu einer Lösung von 3 g (22 mmol) Anthranilsäureamid und 2,23 g (22 mmol) Triethylamin in 12 g Dimethylformamid wird bei Raumtemperatur unter Rühren eine Lösung von 4,33 g (20 mmol) 3,4-Dichlor-isothiazol-5-carbonsäurechlorid in 10 g Dimethylformamid hinzugetropft. Nach beendeter Zugabe wird das Reaktionsgemisch eine Stunde bei 20°C gerührt. Zu der entstandenen Suspension werden bei 0°C unter Rühren 4,3 g (28 mmol) Phosphoroxychlorid hinzugetropft. Das Reaktionsgemisch wird noch 2 Stunden bei 0°C nachgerührt, dann mit 40 ml Wasser versetzt und weitere 15 Minuten gerührt. Der anfallende Feststoff wird abgesaugt, zweimal mit je 20 ml Wasser gewaschen und getrocknet. Das erhaltene Produkt wird 10 Minuten in 15 ml Isopropanol gekocht. Nach dem Abkühlen auf Raumtemperatur wird der anfallende Feststoff abgesaugt, zweimal mit je 5 ml Isopropanol gewaschen und getrocknet. Man erhält auf diese Weise 5,43 g eines Feststoffes, der gemäß HPLC zu 98,6 % aus 3,4-Dichlor-N-(2-cyano-phenyl)-5-isothiazolcarboxamid besteht. Die Ausbeute über 2 Stufen errechnet sich danach zu 89,7 % der Theorie.

### Beispiel 8

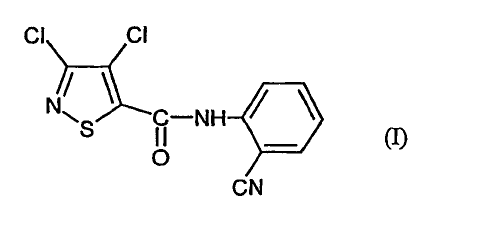

Zu einer Lösung von 6 g (44 mmol) Anthranilsäureamid und 4,46 g (44 mmol) Triethylamin in 24 g Dimethylformamid wird bei Raumtemperatur unter Rühren eine Lösung von 8,66 g (40 mmol) 3,4-Dichlor-isothiazol-5-carbonsäurechlorid in 20 g Dimethylformamid hinzugetropft. Nach beendeter Zugabe wird das Reaktionsgemisch eine Stunde bei 20°C gerührt. In die entstandenen Suspension werden bei 20-25°C unter Rühren 5,6 g (56 mmol) Phosgen eingeleitet. Das Reaktionsgemisch wird noch 2 Stunden bei Raumtemperatur nachgerührt, unter Eiskühlung dann mit 80 ml Wasser versetzt und weitere 15 Minuten gerührt. Der anfallende Feststoff wird abgesaugt, zweimal mit je 40 ml Wasser gewaschen und getrocknet. Das erhaltene Produkt wird 10 Minuten in 30 ml Isopropanol gekocht. Nach dem Abkühlen auf Raumtemperatur wird der anfallende Feststoff abgesaugt, zweimal mit je 10 ml Isopropanol gewaschen und getrocknet. Man erhält auf diese Weise 10,73 g eines Feststoffes, der gemäß ¹H-NMR-Spektrum zu 57 % aus 3,4-Dichlor-N-(2-cyanophenyl)-5-isothiazolcarboxamid besteht. Die Ausbeute über 2 Stufen errechnet sich danach zu 51 % der Theorie.

### Vergleichsbeispiel A

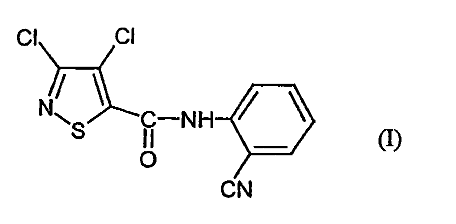

In ein Gemisch aus 20,8 g (0,1725 Mol) 2-Cyano-anilin und 250 ml Pyridin werden bei 5 bis 10°C unter Rühren 38,1 g (0,15 Mol) 3,4-Dichlor-isothiazol-5-carbonsäurechlorid innerhalb von 10 Minuten eingetropft. Nach beendeter Zugabe versetzt man das Reaktionsgemisch mit 70 ml absolutem Tetrahydrofuran und 30 ml Pyridin, lässt auf Raumtemperatur erwärmen und rührt dann 75 Minuten bei Raumtemperatur. Anschließend wird das Reaktionsgemisch unter vermindertem Druck eingeengt. Der verbleibende Rückstand wird mit 800 ml Wasser und 800 ml Essigsäureethylester verrührt. Der in dem Zweiphasen-Gemisch enthaltene Niederschlag wird abfiltriert, mit Essigsäureethylester gewaschen und getrocknet. Man erhält auf diese Weise 31,7 g eines kristallinen Produktes vom Schmelzpunkt 191 bis 193°C.

Aus dem zweiphasigen Filtrat wird die wässrige Phase abgetrennt und zweimal mit je 150 ml Essigsäureethylester extrahiert. Die vereinigten organischen Phasen werden über Natriumsulfat getrocknet und dann unter vermindertem Druck eingeengt. Der verbleibende Rückstand wird mit 100 ml Petrolether und 25 ml Essigsäureethylester verrührt. Der anfallende Feststoff wird abgesaugt, mit Essigsäureethylester nachgewaschen und getrocknet.

Man erhält auf diese Weise insgesamt 40 g (89 % der Theorie) an 3,4-Dichlor-N-(2-cyano-phenyl)-5-isothiazolcarboxamid in Form einer Festsubstanz vom Schmelzpunkt 191-193°C.

## Patentansprüche

1. Verfahren zur Herstellung von 3,4-Dichlor-N-(2-cyano-phenyl)-5-isothiazolcarboxamid der Formel 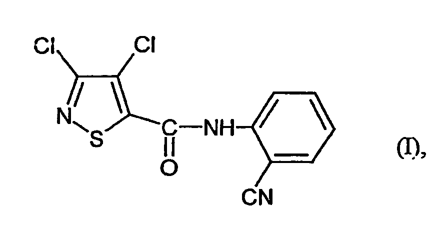 **dadurch gekennzeichnet, dass** man
a) 3,4-Dichlor-isothiazol-5-carbonsäurechlorid der Formel 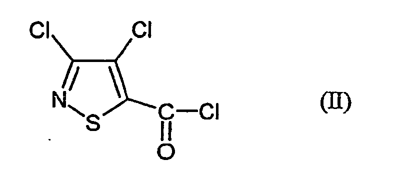 mit Anthranilsäureamid der Formel 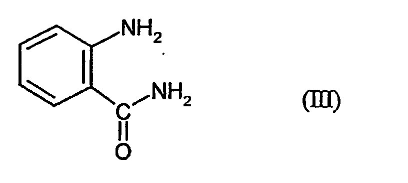 in Gegenwart eines Säureakzeptors und in Gegenwart eines aprotischen Verdünnungsmittels umsetzt und
b) dann das entstandene N-[2-(Aminocarbonyl)-phenyl]-3,4-dichlor-5-isothiazolcarboxamid der Formel 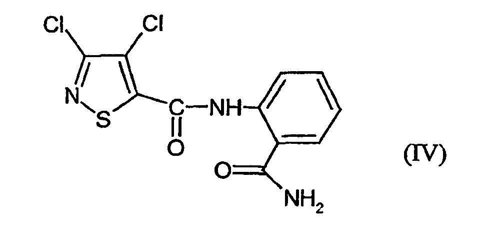 mit einem Dehydratisierungsmittel gegebenenfalls in Gegenwart eines zusätzlichen aprotischen Verdünnungsmittels umsetzt.

2. Verfahren gemäß Anspruch 1, **dadurch gekennzeichnet, dass** man sowohl bei der Durchführung der ersten als auch der zweiten Stufe Dimethylformamid als Lösungsmittel verwendet.

3. Verfahren gemäß Anspruch 1, **dadurch gekennzeichnet, dass** man bei der Durchführung der zweiten Stufe ein Gemisch aus Dimethylformamid und Thionylchlorid, Phosphoroxychlorid, Phosgen oder Chlormethylen-dimethylammoniumchlorid als Dehydratisierungsmittel einsetzt.

4. Verfahren gemäß Anspruch 1, **dadurch gekennzeichnet, dass** man bei der Durchführung der ersten Stufe bei Temperaturen zwischen 0°C und 160°C arbeitet.

5. Verfahren gemäß Anspruch 1, **dadurch gekennzeichnet, dass** man bei der Durchführung der zweiten Stufe bei Temperaturen zwischen -20°C und +80°C arbeitet.

6. Verfahren gemäß Anspruch 1, **dadurch gekennzeichnet, dass** man die Umsetzungen in der ersten und zweiten Stufe als Eintopfreaktion durchführt.

7. N-[2-(Aminocarbonyl)-phenyl]-3,4-dichlor-5-isothiazolcarboxamid der Formel

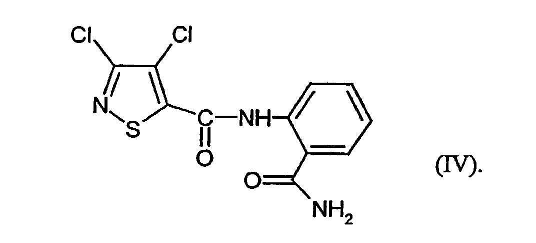

## Claims

1. Process for preparing 3,4-dichloro-N-(2-cyanophenyl)-5-isothiazolecarboxamide of the formula 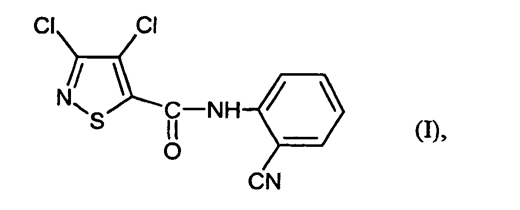 **characterized in that**
a) 3,4-dichloroisothiazole-5-carbonyl chloride of the formula 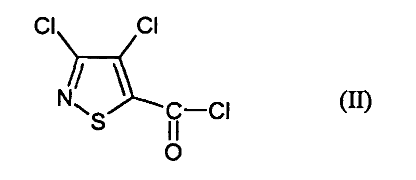 is reacted with anthranilamide of the formula 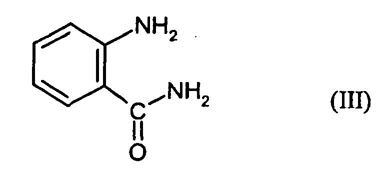 in the presence of an acid acceptor and in the presence of an aprotic diluent and
b) the resulting N-[2-(aminocarbonyl)phenyl]-3,4-dichloro-5-isothiazolecarboxamide of the formula 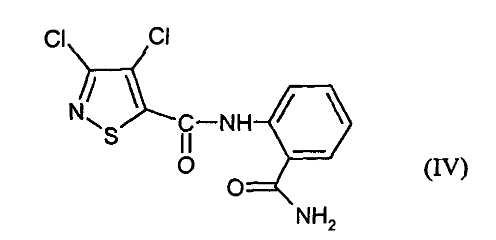 is then reacted with a dehydrating agent, if appropriate in the presence of an additional aprotic diluent.

2. Process according to Claim 1, **characterized in that** the solvent used for carrying out both the first and the second step is dimethylformamide.

3. Process according to Claim 1, **characterized in that** a mixture of dimethylformamide and thionyl chloride, phosphorus oxychloride, phosgene or chloromethylenedimethylammonium chloride as dehydrating agent is used for carrying out the second step.

4. Process according to Claim 1, **characterized in that** the first step is carried out at temperatures between 0°C and 160°C.

5. Process according to Claim 1, **characterized in that** the second step is carried out at temperatures between -20°C and +80°C.

6. Process according to Claim 1, **characterized in that** the first and the second step of the reaction are carried out as a one-pot reaction.

7. N-[2-(Aminocarbonyl)phenyl]-3,4-dichloro-5-isothiazolecarboxamide of the formula

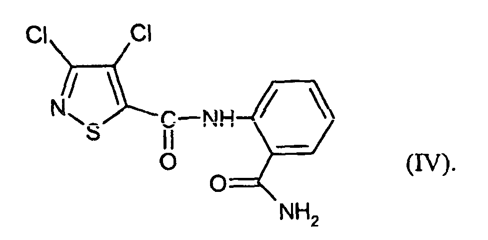

## Revendications

1. Procédé de production de 3,4-dichloro-N-(2-cyanophényl)-5-isothiazole-carboxamide de formule : 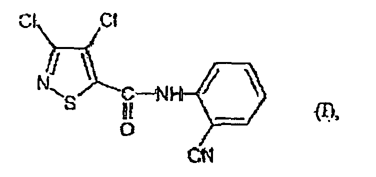 **caractérisé en ce que**
a) on fait réagir le chlorure d'acide 3,4-dichlorisothiazole-5-carboxylique de formule 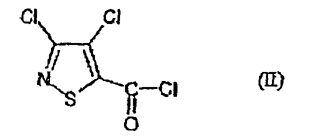 avec l'amide d'acide anthranilique de formule 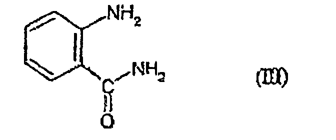 en présence d'un accepteur d'acide et en présence d'un diluant aprotique, puis
b) on fait réagir le N-[2-(aminocarbonyl)-phényl]-3,4-dichloro-5-isothiazole-carboxamide produit de formule : 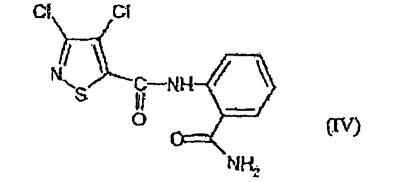 avec un agent déshydratant, éventuellement en présence d'un diluant aprotique supplémentaire.

2. Procédé suivant la revendication 1, **caractérisé en ce qu'**on utilise le diméthylformamide comme solvant tant dans la conduite de la première étape que dans celle de la seconde.

3. Procédé suivant la revendication 1, **caractérisé en ce qu'**on utilise dans la conduite de la seconde étape un mélange de diméthylformamide et de chlorure de thionyle, d'oxychlorure de phosphore, de phosgène ou de chlorure de chlorométhylène-diméthylammonium comme agent déshydratant.

4. Procédé suivant la revendication 1, **caractérisé en ce qu'**on opère à des températures comprises entre 0°C et 160°C dans la conduite de la première étape.

5. Procédé suivant la revendication 1, **caractérisé en ce qu'**on opère à des températures comprises entre -20°C et +80°C dans la conduite de la seconde étape.

6. Procédé suivant la revendication 1, **caractérisé en ce qu'**on conduit les réactions des première et seconde étapes comme réaction en récipient unique.

7. Le N-[2-(aminocarbonyl)-phényl]-3,4-dichloro-5-isothiazole-carboxamide de formule
